# FASCICULE DE BREVET EUROPEEN

(11) **EP 4 370 312 B1**
(45) Date de publication et mention de la délivrance du brevet: **19.11.2025**
(21) Numéro de dépôt: 22754468.1
(22) Date de dépôt: 12.07.2022
(51) Int. Cl.: B29C 65/02, A61M 25/00, B29C 65/00, B29C 65/64, B29C 65/56, B29C 65/18, B29C 65/08, A61M 25/01, A61B 1/005, A61B 1/00, B29L 31/00

(54) **PROCÉDÉ DE FIXATION PAR SOUDURE DE CÂBLES D'ACTIONNEMENT SUR UNE PIÈCE NOTAMMENT DE LA TÊTE DISTALE D'UN DISPOSITIF MÉDICAL**
VERFAHREN ZUR BEFESTIGUNG VON BETÄTIGUNGSKABELN DURCH SCHWEISSEN AN EINER KOMPONENTE, INSBESONDERE AM DISTALEN KOPF EINER MEDIZINISCHEN VORRICHTUNG
METHOD BY WHICH ACTUATING CABLES ARE FIXED BY WELDING TO A COMPONENT IN PARTICULAR OF THE DISTAL HEAD OF A MEDICAL DEVICE

(30) Priorité: 13.07.2021 FR 2107587
(43) Date de publication de la demande: 22.05.2024
(73) Titulaire: Axess Vision Technology, 37300 Joue les Tours (FR)
(72) Inventeur: HALLAUER, Emmanuel, 37190 Saché (FR); LE ROUX, Philippe, 37100 Tours (FR); COURATIN, Jérôme, 37700 Saint-Pierre-des-Corps (FR)
(74) Mandataire: Hindles Limited
(86) Numéro de dépôt international: PCT/FR2022/051403
(87) Numéro de publication internationale: WO 2023/285764

(56) Documents cités:
- WO-A1-2016/188537
- WO-A1-2021/144543
- WO-A1-2021/144544
- JP-A- H10 181 466

## Description

### Domaine Technique

La présente invention concerne le domaine technique de la fixation par soudure, sur une pièce en matériau thermoplastique, de câbles d'actionnement faisant partie d'un mécanisme de commande assurant un mouvement de translation ou de rotation.

L'objet de l'invention trouve une application particulièrement avantageuse mais non exclusivement, pour les dispositifs au sens général permettant d'accéder à l'intérieur d'un corps comme une cavité ou un canal par exemple et elle vise plus précisément les dispositifs médicaux du type cathéter et de préférence, les dispositifs médicaux du type endoscope.

Le dispositif médical du type cathéter ou endoscope selon l'invention est utilisé à des fins de diagnostic, thérapeutiques ou de chirurgie pour l'inspection de toutes les parties internes du corps humain accessibles par les voies naturelles ou artificielles. Par exemple, le dispositif médical selon l'invention peut être utilisé dans le domaine des voies urinaires, des voies gastro-intestinales, du système respiratoire, du système cardiovasculaire, de la trachée, de la cavité du sinus, du système de reproduction de la femme, de la cavité abdominale ou de tout autre partie du corps humain à explorer par une voie naturelle ou artificielle.

### Technique antérieure

D'une manière générale, un endoscope médical comporte, comme décrit par exemple par la demande de brevet WO 2016/188537, une poignée de commande à laquelle est fixé un tube d'insertion. Ce tube comporte une tête distale équipée d'un système de visualisation optique permettant d'éclairer et d'examiner l'organe, la cavité ou le conduit du corps humain. En amont de cette tête distale, le tube d'insertion comporte une structure de flexion ou partie de béquillage permettant l'orientation de la tête distale à l'aide d'un ou plusieurs câbles d'actionnement montés à l'intérieur du tube d'insertion. Chaque câble d'actionnement comporte une extrémité distale fixée à la tête distale et une extrémité proximale sur laquelle agit un mécanisme de commande équipant la poignée pour assurer le coulissement des câbles et par suite, le pliage de cette partie de béquillage afin d'orienter la tête distale.

Classiquement, ce mécanisme de commande comporte un levier de commande agissant sur une pièce pivotante sur laquelle est fixée l'extrémité proximale des câbles d'actionnement. La fixation des câbles d'actionnement sur la pièce pivotante est assurée en enfilant l'extrémité de chaque câble d'actionnement dans des trous aménagés dans la pièce pivotante et en formant une boucle. Un manchon tubulaire enfilé sur le câble d'actionnement est serti sur le câble d'actionnement pour permettre son arrêt en translation. Il est connu des techniques différentes de fixation telles que par vissage notamment. D'une manière générale, il ressort que l'opération de fixation de l'extrémité proximale des câbles d'actionnement sur la pièce pivotante est une opération relativement délicate à mener à bien et susceptible de conduire à une fixation non fiable et/ou à un ancrage ne permettant pas un fonctionnement correct.

Dans l'état de la technique, il est également connu, par le brevet FR 2 477 465 un procédé de fixation d'une plaque en matière plastique à l'extrémité d'un cylindre creux, également en matière plastique à l'extrémité d'un cylindre également en matière plastique. A cet effet, une partie du cylindre creux est chauffé, rabattu vers l'intérieur pour être mis en contact avec la plaque, l'ensemble étant ensuite chauffé et soumis à une pression afin d'être assemblé.

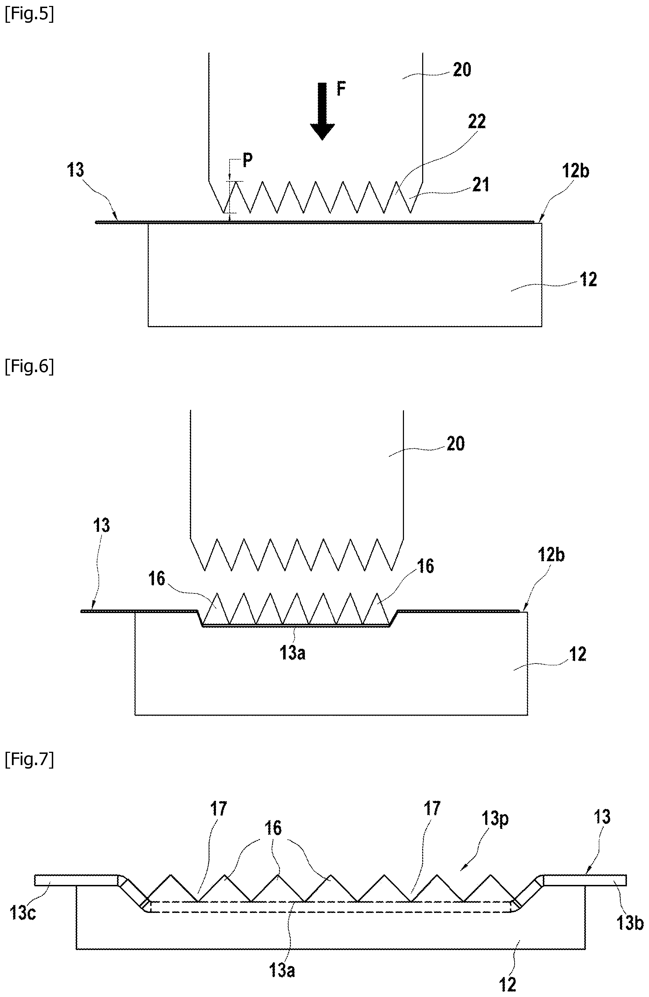

II est également connu par la demande de brevet JP H10181466, un procédé de soudage de câbles électriques sur une pièce thermoplastique présentant des rainures dans chacune desquelles est engagé un câble électrique. Comme illustré à la figure 5, les parois latérales des nervures sont déformées à l'aide d'une tête de soudure comportant une cavité centrale délimitée de part et d'autre, par des dents pour déplacer la matière thermoplastique.

### Exposé de l'invention

La présente invention vise à remédier aux inconvénients de l'état de la technique en proposant une nouvelle technique pour fixer par soudure, sur une pièce en matériau thermoplastique, une extrémité des câbles d'actionnement faisant partie d'un mécanisme de commande, cette nouvelle technique assurant une facilité de mise en œuvre permettant d'en réduire son coût tout en présentant une qualité de fixation optimum durable dans le temps.

Pour atteindre un tel objectif, l'objet de l'invention concerne un procédé visant à fixer sur une pièce, l'extrémité d'un câble d'actionnement faisant partie d'un mécanisme de commande, le procédé comportant les étapes suivantes :
- fournir une pièce faisant partie d'un mécanisme de commande, cette pièce étant pourvue d'au moins une plage en matériau thermoplastique pour la fixation d'une extrémité d'un câble d'actionnement ;
- fournir une tête de soudure dentelée formée de dents alternativement séparées par des creux ;
- positionner l'extrémité d'un câble d'actionnement sur une plage en matériau thermoplastique de la pièce ;
- assurer le chauffage de la plage de la pièce et appliquer la tête de soudure dentelée avec une pression sur l'extrémité du câble d'actionnement pour assurer la pénétration des dents dans la plage en matériau thermoplastique pour déplacer le matériau dans les creux afin d'obtenir au moins un bourrelet de matériau thermoplastique emprisonnant le câble d'actionnement en vue de fixer, par soudage, le câble d'actionnement avec la pièce.

Avantageusement, une tension du câble d'actionnement est appliquée lorsque la pression est exercée sur l'extrémité du câble d'actionnement par la tête de soudure dentelée.

Avantageusement, est fournie une tête de soudure dentelée comportant un nombre de creux compris entre 1 et 15 et de préférence entre 5 et 8 pour déplacer le matériau en vue de l'obtention d'un nombre correspondant de bourrelets de matériau thermoplastique consécutifs.

Par exemple, est fournie une tête de soudure dentelée comportant une série de creux possédant une profondeur comprise entre 0.15 mm et 6 mm pour déplacer le matériau pour obtenir des bourrelets de hauteur déterminée.

Selon une autre caractéristique de l'invention, est fournie une tête de soudure dentelée comportant une série de creux possédant une largeur déterminée pour déplacer le matériau pour obtenir des bourrelets de largeur dépassant de part et d'autre le câble d'actionnement.

Avantageusement, est fournie une tête de soudure dentelée comportant une série de dents présentant chacune une forme triangulaire pour déplacer le matériau pour obtenir des bourrelets de forme correspondante.

De préférence, une partie du câble d'actionnement est positionnée sur la plage en matériau thermoplastique, à l'aide de structures de positionnement aménagées sur ladite plage.

Avantageusement, un soudage par ultrasons assure la fixation du câble d'actionnement à la pièce.

Selon un autre exemple de mise en œuvre, un bouterollage à chaud et par pression assure la fixation du câble d'actionnement à la pièce.

Selon une application préférée, on fournit en tant que pièce , une pièce pivotante faisant partie d'un mécanisme de commande permettant d'orienter la tête distale d'un dispositif médical, cette pièce pivotante étant pourvue de deux plages en matériau thermoplastique pour la fixation chacune d'une extrémité proximale d'un câble d'actionnement.

Avantageusement, la pièce d'un mécanisme de commande pourvue d'au moins une plage de fixation en matériau thermoplastique présente au moins un bourrelet en matériau thermoplastique emprisonnant une partie d'ancrage d'un câble d'actionnement enfoncée dans la pièce en étant située en retrait par rapport aux parties du câble d'actionnement situées de part et d'autre de la partie d'ancrage.

La pièce pourvue de deux plages en matériau thermoplastique présente chacune une série de bourrelets en matériau thermoplastique emprisonnant chacune une partie d'ancrage d'un câble d'actionnement enfoncée dans la pièce en étant située en retrait par rapport aux parties du câble d'actionnement situées de part et d'autre de la partie d'ancrage.

La pièce comporte des bourrelets en matériau thermoplastique emprisonnant le câble d'actionnement et en étant situés à proximité de l'entrée d'une rainure de guidage pour le câble d'actionnement.

Avantageusement, la pièce comporte des bourrelets se présentant sous la forme de dentelures successives recouvrant le câble d'actionnement et séparées entre elles par des dégagements dans lesquels affleure le câble d'actionnement.

Selon une caractéristique avantageuse de réalisation, la pièce est pivotante et fait partie d'un mécanisme de commande permettant d'orienter la tête distale d'un dispositif médical.

De préférence, la pièce se présente sous la forme d'un disque ou d'une bague annulaire pour former une poulie d'actionnement.

L'objet de l'invention vise avantageusement une poignée de commande pour un dispositif médical comportant un mécanisme de commande permettant d'orienter la tête distale du dispositif médical, le mécanisme de commande comportant une pièce conforme à l'invention.

Diverses autres caractéristiques ressortent de la description faite ci-dessous en référence aux dessins annexés qui montrent, à titre d'exemples non limitatifs, des formes de réalisation de l'objet de l'invention.

### Brève description des dessins

[Fig. 1]La Figure 1 est une vue schématique d'un dispositif médical du type cathéter ou endoscope au sens général comportant une poignée de commande pourvue d'un mécanisme de commande permettant d'orienter la tête distale du dispositif médical.
[Fig. 2]La Figure 2 est une vue en perspective montrant une pièce pivotante réalisée sous la forme d'une poulie pourvue de deux câbles d'actionnement.
[Fig. 3]La Figure 3 est une vue en perspective montrant une pièce pivotante réalisée sous la forme d'une poulie sur laquelle sont destinés à être fixés deux câbles d'actionnement.
[Fig. 4]La Figure 4 est une vue en perspective montant un exemple de réalisation d'une tête de soudure destinée à fixer un câble d'actionnement sur une pièce pivotante.
[Fig. 5]La Figure 5 est une vue schématique montrant, avant fixation par une tête de soudure, le positionnement d'un câble d'actionnement sur une pièce.
[Fig. 6]La Figure 6 est une vue schématique montrant, après fixation par une tête de soudure, l'ancrage d'un câble d'actionnement sur une pièce.
[Fig. 7]La Figure 7 est une vue schématique en coupe élévation, montrant après fixation par une tête de soudure, l'ancrage d'un câble d'actionnement sur une pièce.
[Fig. 8]La Figure 8 est une vue schématique de dessus, montrant après fixation par une tête de soudure, l'ancrage d'un câble d'actionnement sur une pièce.
[Fig. 9]La Figure 9 est une vue schématique de dessus, montrant après fixation par une tête de soudure, un autre exemple de réalisation de l'ancrage d'un câble d'actionnement sur une pièce.

### Description des modes de réalisation

L'objet de l'invention concerne une nouvelle technique pour fixer sur une pièce, l'extrémité de câbles d'actionnement faisant partie d'un mécanisme de commande assurant un mouvement de translation ou de rotation. L'objet de l'invention trouve des applications dans de nombreux domaines techniques tels que par exemple, les machines, les véhicules ou les dispositifs médicaux.

La présente invention trouve une application particulièrement avantageuse pour la fixation des câbles d'actionnement de la structure de flexion permettant d'orienter la tête distale de dispositifs médicaux du type cathéter ou endoscope. La description qui suit illustre à titre d'exemple préféré, la mise en œuvre de l'invention, pour un dispositif médical 1 du type endoscope ou cathéter au sens général conçu pour accéder à l'intérieur d'un corps comme une cavité ou un canal par exemple.

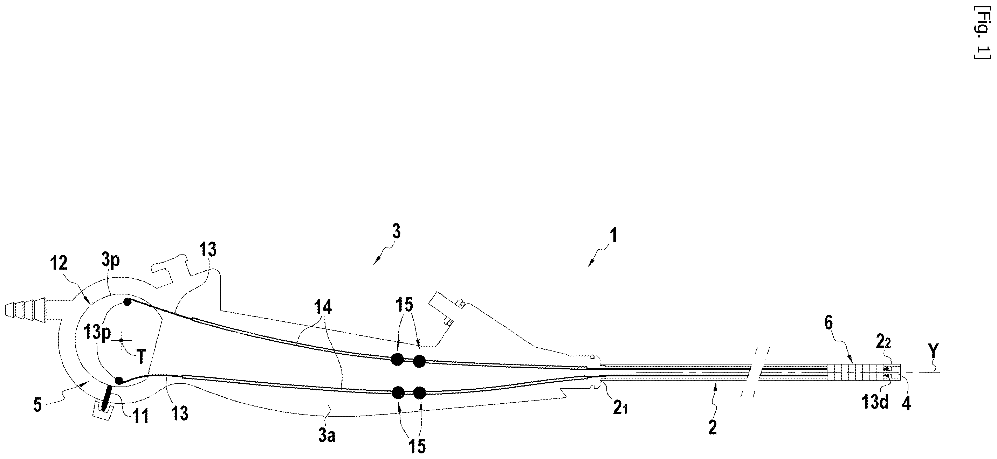

Tel que cela ressort plus précisément de la Figure 1, un dispositif médical 1 du type endoscope ou cathéter comporte un tube d'insertion 2 présentant d'un côté, une partie proximale 2₁ reliée au boîtier 3a d'une poignée de commande 3 et du côté opposé, une partie distale 2₂, qui est équipée d'une tête distale 4. Le tube d'insertion 2 est fixé de manière temporaire ou définitive sur le boîtier de la poignée de commande 3. Ce tube d'insertion 2 qui présente une longueur et une flexibilité plus ou moins importante est destiné à être introduit dans une voie d'accès naturelle ou artificielle en vue d'effectuer diverses opérations ou fonctions à des fins thérapeutiques, chirurgicales ou de diagnostic. Le tube d'insertion 2 est réalisé en un matériau semi-rigide et présente une longueur adaptée à la longueur du conduit à inspecter et pouvant être comprise entre 5 cm et 2 m. Le tube d'insertion 2 présente diverses formes de section transversale telles que carrée, ovale ou circulaire. Ce tube d'insertion 2 qui est en contact avec les tissus, les organes humains ou des appareillages médicaux (trocarts ou sondes), relève essentiellement d'un usage unique ou multiple d'un patient voire d'un usage réutilisable après décontamination, désinfection ou stérilisation.

Selon un exemple préféré de réalisation, le dispositif médical 1 conforme à l'invention est un endoscope comportant un système de vision apte à éclairer et à ramener une image de la partie distale du tube d'insertion 2. L'endoscope comporte ainsi un système de vision monté à l'intérieur de la poignée de commande 3 et pénétrant à l'intérieur du tube d'insertion 2 jusqu'à la tête distale 4.

De manière classique, le dispositif médical 1 comporte également un mécanisme de commande 5 permettant d'orienter la tête distale 4 par rapport à l'axe longitudinal Y du tube d'insertion 2. A cet effet, le tube d'insertion 2 comporte en amont de la tête distale 4, une partie de flexion, de pliage ou de béquillage 6 permettant l'orientation de la tête distale 4 par rapport à l'axe longitudinal Y du tube d'insertion 2. Cette partie de flexion, de pliage ou de béquillage 6 peut être réalisée de toute manière appropriée pour assurer la flexion de la tête distale 4 par rapport à l'axe longitudinal Y du tube d'insertion 2. Par exemple, cette partie de flexion, de pliage ou de béquillage 6 peut être réalisée par un ressort ou par des vertèbres tubulaires articulées entre elles.

Le mécanisme de commande 5 peut être réalisé de toute manière appropriée de manière que la tête distale 4 puisse être déplacée entre une position de repos dans laquelle le tube d'insertion 2 est rectiligne et une position béquillée dans laquelle la partie de béquillage 6 est courbée. A titre d'exemple non limitatif, le mécanisme de commande 5 peut correspondre au mécanisme de commande décrit dans le brevet FR 3 047 887. A cet effet, le mécanisme de commande 5 comporte un levier de commande manuelle 11 accessible de l'extérieur du boîtier 3a de la poignée de commande. Ce levier de commande 11 agit directement ou indirectement sur au moins une pièce 12 de fixation ou de support de manière à entraîner la rotation de la pièce autour d'un axe transversal de rotation T. Cette pièce 12 est reliée à la tête distale 4 de sorte qu'une rotation de la pièce 12 entraîne la flexion de la tête distale 4.

Ainsi, à la suite de l'application d'un effort manuel sur le levier de commande 11, il est obtenu la flexion de la tête distale 4. Selon un exemple de réalisation illustré à la Figure 1, le levier de commande 11 est un levier guidé en rotation sur une plage angulaire limitée, selon un axe transversal de rotation T. Il est à noter que le levier de commande peut être réalisé de manière différente comme sous la forme d'un poussoir guidé en déplacement linéaire et sollicité en rappel élastique pour agir sur la pièce par l'intermédiaire d'un système de transformation du mouvement linéaire du poussoir en un mouvement de rotation de la pièce 12.

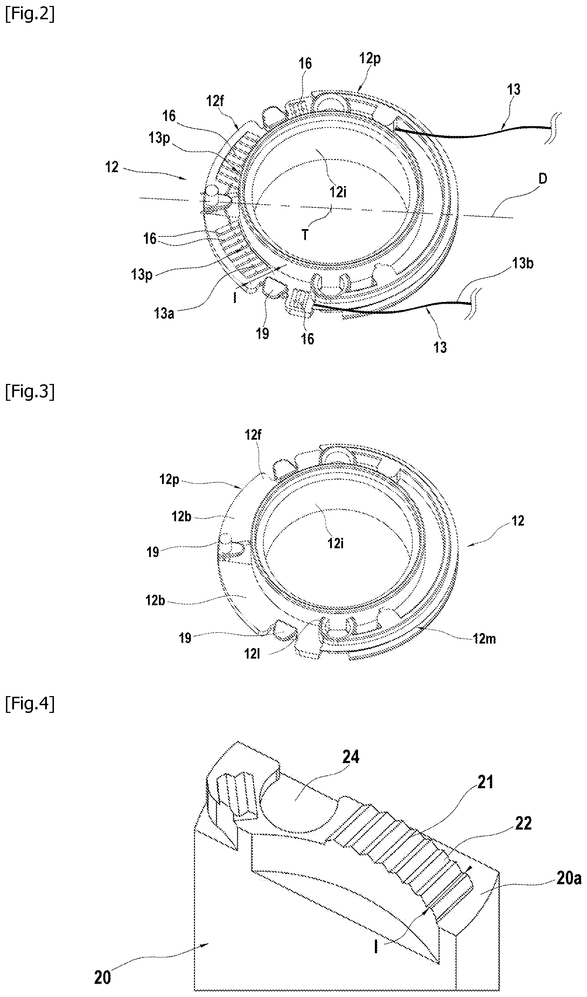

Cette pièce 12 est dans l'exemple de réalisation, guidée en rotation par un palier annulaire 3p aménagé dans le boîtier ou par une pièce rapportée dans le boîtier 3a. La pièce 12 est guidée par une liaison pivot de manière à présenter uniquement un mouvement de rotation autour de l'axe transversal de rotation T. La pièce 12 peut être réalisée de différentes manières en considérant que le mouvement de rotation de la pièce est limité à moins d'un tour et en particulier, inférieur à un tiers d'un tour. Selon l'exemple de réalisation préféré illustré aux Figures 2 et 3, la pièce 12 est une pièce pivotante réalisée sous la forme d'une bague annulaire plate délimitée par un bord périphérique circulaire 12p et un bord intérieur circulaire 12i. Cette pièce 12 comporte ainsi deux faces planes ou plates annulaires 12f s'étendant de part et d'autre de la pièce. Cette pièce 12 forme une poulie comme cela sera mieux compris dans la suite de la description. Bien entendu, la pièce 12 peut être réalisée de manière différente comme sous la forme d'une semi-bague ou d'une portion d'anneau par exemple. De même, la pièce 12 peut être réalisée sous la forme d'un disque plein ou d'une came pour faire varier la flexion de manière non linéaire.

Les Figures 2 et 3 montrent un exemple de réalisation de la pièce sous la forme d'une bague annulaire 12 qui n'est pas munie du levier de commande 11. Par exemple, le levier de commande 11 est apte à être rapporté sur une face plane 12f par tous moyens appropriés comme par l'emboitement d'un téton dans un logement 12l de la bague annulaire. Bien entendu, le levier de commande 11 et la bague annulaire 12 peuvent aussi être fabriqués en une seule pièce.

Au moins un et dans l'exemple illustré, deux câbles d'actionnement 13 sont fixés à la pièce 12. Ces câbles d'actionnement 13 sont montés à l'intérieur du tube d'insertion 2 pour être fixés à la tête distale 4. Les extrémités proximales 13p des câbles d'actionnement 13 sont fixées à la pièce 12 tandis que les extrémités distales 13d des câbles d'actionnement 13 sont fixées à la tête distale 4. Dans l'exemple illustré, les extrémités proximales 13p des câbles d'actionnement 13 sont fixées de façon symétriquement opposée sur la pièce 12 par rapport à un plan diamétral.

Ce mécanisme de commande 5 est adapté pour assurer par exemple le déplacement gauche-droit ou haut-bas de la tête distale 4. Bien entendu, le mécanisme de commande 5 peut être adapté pour assurer le déplacement gauche-droit et haut-bas de la tête distale 4 à l'aide de trois ou de quatre câbles d'actionnement 13. Dans l'exemple illustré, le levier de commande 11 a une course en rotation mais comme déjà décrit, il est possible que le levier de commande 11 possède un mouvement de translation entraînant la rotation de la pièce 12 par l'intermédiaire d'un système de transformation de mouvement.

Bien entendu, les câbles d'actionnement 13 sont réalisés de toute manière appropriée pour assurer la fonction de transmission de mouvement conduisant à la flexion de la tête distale 4. Ainsi, ces câbles 13 peuvent être réalisés par exemple par des tiges, fils, tresses, filaments, torons ou chaînes, réalisés en un matériau métallique ou polymère par exemple. Typiquement, les câbles d'actionnement 13 sont réalisés par des tresses en acier inoxydable selon un diamètre compris entre 0,05 mm et 4 mm.

Chaque câble d'actionnement 13 est monté à l'intérieur d'une gaine de support 14 destinée à être fixée au boîtier 3a de la poignée de commande 3, à l'aide d'un système de fixation 15 de tous types connus en soi. La gaine de support 14 assure ainsi le guidage en coulissement du câble d'actionnement 13 lors des opérations de béquillage de la tête distale 4. Le câble d'actionnement 13 se déplace ainsi en translation par rapport à la gaine de support 14 qui est montée solidaire de la poignée de commande 3.

Conformément à l'invention, la fixation des câbles d'actionnement 13 avec la pièce 12 est effectuée par soudage. Plus précisément, les câbles d'actionnement 13 sont fixés dans l'exemple illustré, par leur partie proximale 13p sur l'une et/ou l'autre des faces planes 12f de la pièce 12. A cet effet, il est considéré que la pièce 12 présente par ses faces planes 12f, pour chaque câble d'actionnement 13, une plage de fixation 12b qui dans l'exemple illustré est plane. Les deux plages de fixation 12b sont situées de manière symétriquement opposées par rapport à un plan diamétral D de la pièce 12 passant par l'axe transversal de rotation T. De préférence, les deux plages de fixation 12b sont situées en dehors du plan diamétral D pour permettre aux câbles d'actionnement 13 d'être guidés sur une plage angulaire importante de la pièce comme cela sera expliqué dans la suite de la description. Bien entendu, il peut être envisagé que chaque face plane 12f de la pièce 12 comporte une plage de fixation 12b.

Conformément à l'invention, la pièce 12 comporte pour chaque câble d'actionnement 13, une plage de fixation 12b présentant, après l'opération de soudage, au moins un bourrelet 16 et de préférence une série de bourrelets 16 emprisonnant un câble d'actionnement 13 ancré à la pièce 12. Chaque bourrelet 16 est une excroissance ou une protubérance de matériau réalisée par l'opération de soudage et provenant du matériau constitutif de la pièce 12. Chaque bourrelet 16 s'étend en saillie par rapport à cette plage de fixation 12b en s'élevant au-dessus du câble d'actionnement 13. Avantageusement, les bourrelets 16 sont aménagés consécutivement le long d'une partie du câble d'actionnement 13. L'extrémité proximale 13p de chaque câble d'actionnement 13 est fixée par soudage, par l'intermédiaire des bourrelets 16 de manière sûre et efficace afin que le mouvement de la pièce 12 puisse être transmis à la tête distale 4.

Dans l'exemple illustré sur les Figures, le mécanisme de commande 5 comporte deux câbles d'actionnement 13 fixés chacun à la pièce 12 à l'aide des bourrelets 16 de fixation conformes à l'invention. Bien entendu, un seul câble d'actionnement 13 peut être fixé à la pièce 12.

Tel que cela ressort plus précisément des Figures 2 et 7, l'extrémité proximale 13p de chaque câble d'actionnement 13 comporte ainsi une partie d'ancrage 13a emprisonnée par les bourrelets 16 et se prolongeant d'un côté, par une partie d'actionnement 13b qui se termine par l'extrémité distale 13d fixée à la tête distale 4. De préférence, la partie d'ancrage 13a du câble d'actionnement 13 se prolonge à l'opposé de sa partie d'actionnement 13b, par une partie d'extrémité ou chute 13c.

Selon une variante avantageuse de réalisation, la pièce 12 est aménagée pour permettre le positionnement et le guidage du câble d'actionnement 13. Ainsi, la pièce 12 comporte une rainure circulaire 12m centrée sur l'axe de rotation T de la pièce et aménagée dans la face plane 12f en périphérie de la pièce 12. Au cours du pivotement de la pièce 12, chaque câble d'actionnement 13 se trouve ainsi guidé par la rainure circulaire 12m.

Avantageusement, la rainure circulaire 12m est aménagée avec une profondeur adaptée pour loger les câbles d'actionnement 13 qui sont maintenus en contact de la pièce en raison de la tension imposée aux câbles d'actionnement 13 lorsque la pièce 12 est montée dans le boîtier 3a de la poignée de commande. Cette pièce 12 munie des câbles d'actionnement forme une poulie d'actionnement apte à faire partie du mécanisme d'actionnement 5 pour un endoscope dans l'exemple décrit.

Selon une caractéristique avantageuse de réalisation, la pièce 12 comporte des bourrelets 16 en matériau thermoplastique emprisonnant le câble d'actionnement 13 en étant située à proximité de l'entrée de la rainure de guidage 12m pour le câble d'actionnement. De tels bourrelets 16 permettent le bon positionnement des câbles d'actionnement dans les rainures de guidage 12m. Tel que cela ressort plus précisément de la figure 2, la pièce 12 comporte deux séries de bourrelets 16 consécutifs compte tenu de la configuration de la pièce mais il clair que les bourrelets 16 peuvent être aménagées sur la pièce selon une ou plusieurs séries.

Après la fixation du câble d'actionnement 13 à la pièce 12, les bourrelets 16 se présentent comme cela ressort plus précisément des Figures 2 et 7, sous la forme de dentelures successives recouvrant le câble d'actionnement 13 et séparées entre elles par des dégagements 17 dans lesquels affleure le câble d'actionnement 13. Le câble d'actionnement 13 est enfoncé dans la pièce 12 au niveau des dentelures. En d'autres termes, la partie d'ancrage 13a du câble d'actionnement 13 est enfoncée dans la pièce 12 dans le sens où la partie d'ancrage 13a est située en retrait par rapport à la partie d'actionnement 13b et à la partie d'extrémité ou chute 13c. Comme illustré à la Figure 7, la partie d'actionnement 13b et la partie d'extrémité ou chute 13c s'étendent dans un plan passant par la plage de fixation 12b et en retrait duquel s'étend la partie d'ancrage 13a du câble d'actionnement 13. Par exemple, la partie d'ancrage 13a du câble d'actionnement 13 s'étend sensiblement dans un plan situé en retrait du plan contenant la partie d'actionnement 13b et la partie d'extrémité ou chute 13c.

Avantageusement, les bourrelets 16 présentent une largeur l dépassant de part et d'autre du câble d'actionnement 13, comme cela ressort plus précisément de la Figure 2. Ainsi, la largeur des bourrelets 16 prise selon une direction perpendiculaire à la direction d'extension du câble d'extension 13 est supérieure au diamètre du câble d'actionnement 13 pour pouvoir dépasser de chaque côté du câble d'actionnement.

Les bourrelets 16 coopèrent avec le câble d'actionnement 13 pour lier le câble d'actionnement 13 à la pièce 12. En d'autres termes, les bourrelets 16 sont en contact intime avec le câble d'actionnement 13 tout en étant apte à s'insérer dans l'épaisseur du câble d'actionnement à partir de sa surface extérieure. Les bourrelets 16 assurent en combinaison le blocage en translation du câble d'actionnement 13.

Selon une caractéristique de l'invention, le câble d'actionnement 13 est fixé par soudage à la pièce 12. Selon une caractéristique de l'invention, les bourrelets 16 sont en matériau thermoplastique c'est-à-dire en un matériau susceptible d'être ramolli par chauffage, au-dessus d'une certaine température et durci par refroidissement. Par exemple, le matériau dans lequel sont réalisés les bourrelets 16 relève de la catégorie des polymères thermoplastiques semi-cristallins ou amorphes. Typiquement, les bourrelets 16 sont par exemple en ABS (acrylonitrile butadiène styrène), PP (polyprolylène), POM (polyoxyméthylène), polyamide, polyuréthane, PE (polyéthylène), PS (polystyrène), ou PA (Polyamide), PE (Polyéthylène, SAN (Poly(styrène/acrylonitrile), PEEK (Polyétheréthercétone), PPS (Polysulfure de phénylène), ou un mélange quelconque de ces polymères.

Comme les bourrelets 16 sont formés à partir du matériau constitutif de la plage de fixation 12b, la plage de fixation 12b de la pièce 12 est réalisée en un matériau thermoplastique. Selon une variante préférée de réalisation, la pièce 12 et la plage de fixation sont réalisés selon un même matériau thermoplastique. Typiquement, la pièce 12 est fabriquée selon la technique de moulage au cours de laquelle la pièce 12 est formée en étant pourvue de la plage de fixation 12b. Bien entendu, il pourrait être envisagé de rapporter la plage de fixation 12b en matériau thermoplastique sur une pièce 12 réalisée en tous types de matériaux.

La fixation d'un câble d'actionnement 13 sur la pièce 12 est réalisée par le procédé décrit ci-après en relation plus précisément des Figures 5 et 6.

La première étape du procédé de fixation consiste à fournir une pièce 12 pour un mécanisme de commande 5, pourvue d'au moins une plage 12b en matériau thermoplastique pour la fixation de l'extrémité proximale 13p du câble d'actionnement 13. Dans l'exemple illustré à la Figure 2, la pièce 12 comporte pour les deux câbles d'actionnement 13, deux plages de fixation 12b de surface plane.

Selon une caractéristique avantageuse de réalisation, la pièce 12 est pourvue d'une ou de plusieurs de structures 19 assurant le positionnement du câble d'actionnement 13 sur la plage de fixation 12b. Ces structures d'aide au positionnement 19 peuvent se présenter sous toutes formes appropriées pour positionner et maintenir le câble d'actionnement préalablement à l'opération de soudure proprement dite. Comme illustré plus précisément à la Figure 3, les structures de positionnement 19 peuvent se présenter sous la forme de tétons ou de languettes.

Le procédé de fixation consiste ainsi à positionner l'extrémité proximale 13p de chaque câble d'actionnement 13 sur une plage de fixation 12b en matériau thermoplastique de la pièce 12. L'étape de mise en place du câble d'actionnement 13 sur la pièce peut être réalisée manuellement ou automatiquement à l'aide d'un bras robotisé.

Le procédé de fixation consiste également à fournir une tête de soudure dentelée 20 formée de dents 21 alternativement séparées par des creux 22. Il doit être compris que le nombre de dents 21 et de creux 22 est choisi en fonction du nombre de bourrelets 16 souhaités. En effet, comme cela sera expliqué en détail dans la suite de la description, chaque bourrelet 16 est formé par le matériau présent dans un creux 22 situé entre deux dents 21 consécutives. La tête de soudure dentelée 20 comporte un nombre de creux 22 compris entre 1 et 15, de préférence entre 3 et 12 et selon une variante avantageuse de réalisation, entre 5 et 8.

Tel que cela ressort plus particulièrement des Figures 4 et 5, les dents 21 et les creux 22 sont aménagées à la partie terminale de la tête de soudure dentelée 20. La tête de soudure dentelée 20 présente une face transversale d'extrémité 20a à partir de laquelle s'élèvent en saillie les dents 21. Dans l'exemple de réalisation illustré sur les dessins, la tête de soudure dentelée 20 présente un dégagement 24 laissant subsister d'un côté, une première série de dents 21 et de creux 22 et de l'autre côté, une deuxième série de dents 21 et de creux 22. Le dégagement 24 est adapté pour réaliser une structure de positionnement 19.

Selon une caractéristique avantageuse de réalisation, la tête de soudure dentelée 20 comporte une série de dents 21 présentant une forme déterminée pour déplacer le matériau pour obtenir des bourrelets 16 de forme correspondante. Les dents 21 peuvent prendre toutes les formes adaptées pour réaliser des bourrelets 16 de matière. Selon une caractéristique avantageuse de réalisation illustrée sur les dessins, les dents 21 présentent une forme triangulaire. Chaque dent 21 possède ainsi une section droite transversale triangulaire. Comme illustré sur les dessins, les dents 21 présentent perpendiculairement à leur section droite transversale, une forme droite c'est-à-dire que les dents présentent des arêtes rectilignes sur toute leur largeur.

Selon une caractéristique avantageuse de réalisation, la tête de soudure dentelée 20 comporte une série de creux 22 possédant une profondeur p comprise entre 0.15 mm et 6 mm pour déplacer le matériau pour obtenir des bourrelets 16 de hauteur déterminée. Comme illustré par la Figure 5, la profondeur p d'un creux 22 correspond à la distance prise selon une direction perpendiculaire à la face transversale d'extrémité 20a, entre le fond du creux 22 et l'extrémité d'une dent 21 voisine.

Selon une autre caractéristique avantageuse de réalisation, la tête de soudure dentelée 20 comporte une série de creux 22 et de dents 21 possédant une largeur l déterminée pour obtenir des bourrelets 16 de largeur dépassant de part et d'autre le câble d'actionnement. La largeur l des creux 22 et des dents 21 qui est prise perpendiculairement à la section droite transversale des dents 21 est comprise entre 0.2 et 12 mm.

Le procédé de fixation consiste à assurer le chauffage de la plage de fixation 12b et à appliquer la tête de soudure dentelée 20 avec une pression sur l'extrémité proximale 13p du câble d'actionnement 13 pour assurer la pénétration des dents 21 dans la plage en matériau thermoplastique. La pénétration de la tête de soudure dentelée 20 permet de déplacer à l'aide des dents 21, le matériau dans les creux 22 afin d'obtenir une série de bourrelets 16 consécutifs de matériau thermoplastique emprisonnant le câble d'actionnement 13 afin de le fixer, par soudage, avec la pièce 12. Le niveau de pénétration de la tête de soudure dentelée 20 dans la pièce 12 détermine le volume de matériau déplacé destiné à pénétrer dans les creux 22. En fonction du volume de matériau déplacé, les bourrelets 16 prennent plus ou moins la forme des creux 22. Dans l'exemple illustré sur les dessins, les bourrelets 16 présentent une section droite transversale triangulaire.

Tel que cela ressort de la Figure 5, au moins l'effort de pression est exercé à l'aide d'une tête de soudure dentelée 20 adaptée au procédé de soudage mis en œuvre. En effet, la chaleur peut être apportée par la tête de soudure dentelée 20 et/ou par un dispositif indépendant de la tête de soudure dentelée 20. Dans le même sens, la chaleur peut être apportée avant l'application de l'effort de pression ou simultanément à l'application de l'effort de pression. Cette étape de soudure du câble d'actionnement 13 peut être réalisée manuellement ou automatiquement à l'aide d'un bras robotisé. Par exemple, les paramètres de déplacement de la tête de soudure dentelée 20 ainsi que les paramètres de température qui peuvent être définis pour obtenir un soudage efficace sont aptes à être reproduits.

Selon une caractéristique avantageuse de mise en œuvre, une tension du câble d'actionnement 13 est appliquée lorsque la pression est exercée sur l'extrémité proximale 13p du câble d'actionnement 13 par la tête de soudure dentelée 20. La tension sur le câble d'actionnement est exercée par tous systèmes appropriés. Cette tension de précontrainte appliquée sur le câble d'actionnement permet d'éviter des jeux et assure d'obtenir un béquillage répondant et fonctionnel.

L'apport de chaleur au niveau de l'extrémité libre de la plage de fixation 12b conduit à son ramollissement de sorte que le matériau est déplacé sous l'effet de l'application d'une pression selon un sens de rapprochement de la tête de soudure dentelée 20 en direction du câble d'actionnement 13, représenté par la flèche F sur la Figure 5. La direction de déplacement de la tête de soudure dentelée 20 s'établit avantageusement perpendiculairement au plan d'extension de la plage de fixation 12b. Avantageusement, la tête de soudure dentelée 20 est déplacée par rapport à la pièce 12. Bien entendu, il peut être envisagé de déplacer la pièce 12 par rapport à la tête de soudure dentelée 20.

La fixation du câble d'actionnement 13 à la pièce 12 est réalisée par la mise en œuvre d'un procédé de bouterollage à chaud (laser, induction, air chaud, infra-rouge par exemple) ou un procédé de soudage vibratoire par friction (orbitale, longitudinale ou axiale).

Selon un exemple préféré de mise en œuvre, la fixation du câble d'actionnement 3 à la pièce 12 est réalisée par la mise en œuvre d'un procédé de soudage par ultrasons. Selon cet exemple de réalisation, la pièce 12 est par exemple positionnée sur une enclume et la tête de soudure dentelée 20 telle qu'une sonotrode vibratoire est appliquée sur l'extrémité proximale 13p du câble d'actionnement 13 supportée par la plage de fixation 12b en étant déplacée de quelques mm vers l'intérieur de la plage de fixation pour déplacer le matériau de cette partie de la pièce 12.

Le procédé selon l'invention permet d'obtenir une pièce 12 munie de bourrelets 16 assurant la fixation de câbles d'actionnement 13 de la tête distale 4 d'un dispositif médical 1 du type endoscope ou cathéter. Cette pièce 12 fait partie d'un mécanisme de commande 5 monté dans une poignée de commande pour un dispositif médical 1 du type endoscope ou cathéter.

Selon la description qui précède, l'objet de l'invention concerne la fixation des câbles d'actionnement 13 de la structure de flexion permettant d'orienter la tête distale de dispositifs médicaux du type cathéter ou endoscope. Dans l'exemple de réalisation illustré, les câbles d'actionnement 13 sont fixés sur la poulie faisant partie du mécanisme de commande. Il est à noter que le câble d'actionnement 13 peut être fixé à un manchon en matériau thermoplastique destiné à être monté par exemple sur la poulie faisant partie du mécanisme de commande.

Dans la description qui précède, le câble d'actionnement 13 est fixé à une pièce 12 par son extrémité proximale 13p. Il est clair que le câble d'actionnement 13 peut être fixé à une pièce 12 par son extrémité distale ou en par toute autre de ses parties.

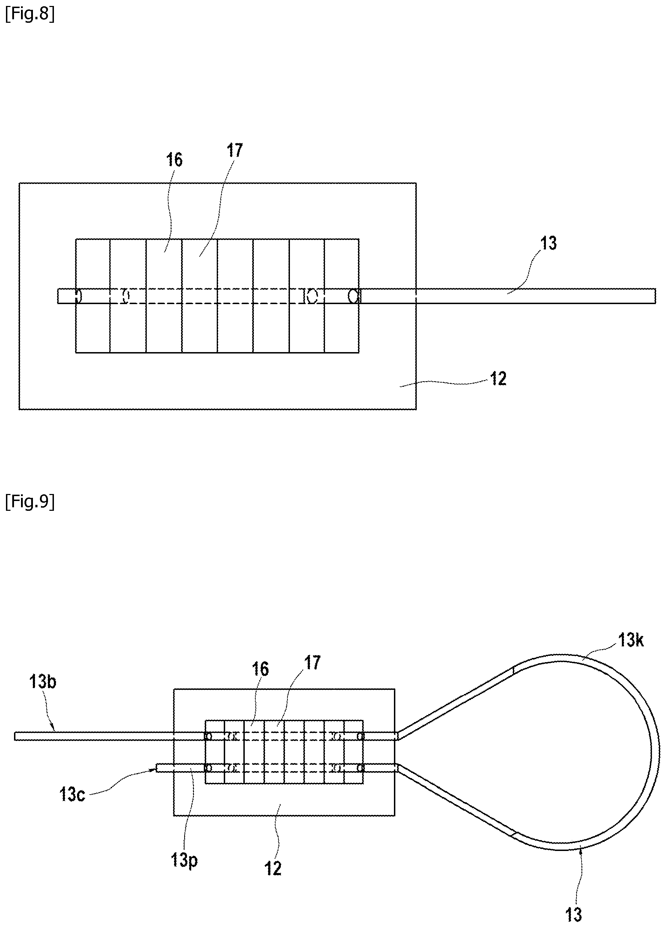

La figure 9 illustre une variante de réalisation de la fixation d'un câble d'actionnement 13 sur une pièce 12 en matériau thermoplastique visant à réaliser une boucle 13k à l'extrémité du câble d'actionnement 13. A cet effet, le câble d'actionnement 13 est positionné sur la pièce 12 pour former en dehors de la pièce 12, une boucle 13k destinée par exemple à entourer une pièce 12 cylindrique. L'extrémité proximale 13p du câble d'actionnement 13 est repliée pour être positionnée à proximité d'une portion du câble d'actionnement 13, par exemple parallèlement l'une à l'autre. Dans cet exemple, cette portion du câble d'actionnement 13 et l'extrémité proximale 13p du câble d'actionnement 13 sont fixées par une série de bourrelets 16 communs. En d'autres termes, la fixation du câble est assurée à l'aide d'une tête de soudure dentelée dont la largeur des dents 21 assure l'ancrage simultané des deux portions du câble d'actionnement 13. Bien entendu, chaque portion du câble d'actionnement 13 peut être fixée à l'aide de bourrelets 16 distincts.

Il ressort de la description qui précède que l'objet de l'invention est adapté pour assurer la fixation par soudage, sur une pièce 12 en matériau thermoplastique, de câbles d'actionnement 13 faisant partie d'un mécanisme de commande d'un dispositif médical au sens général. L'objet de l'invention trouve d'autres applications pour assurer la fixation par soudage, sur une pièce 12 en matériau thermoplastique, de câbles d'actionnement 13 produisant un mouvement de rotation ou de translation. Tel est le cas par exemple, de la fixation des câbles d'actionnement 13 avec des pièces 12 faisant partie du mécanisme de frein d'une bicyclette ou d'un mécanisme d'accélération d'un véhicule.

## Revendications

1. Procédé de fixation d'une extrémité d'un câble d'actionnement (13) faisant partie d'un mécanisme de commande (5), le procédé comportant les étapes suivantes :
- fournir une pièce (12) faisant partie d'un mécanisme de commande, cette pièce (12) étant pourvue d'au moins une plage (12b) en matériau thermoplastique pour la fixation d'une extrémité (13p) d'un câble d'actionnement (13) ;
- fournir une tête de soudure dentelée (20) formée de dents alternativement séparées par des creux ;
- positionner l'extrémité (13p) d'un câble d'actionnement (13) sur une plage en matériau thermoplastique de la pièce ;
- assurer le chauffage de la plage de la pièce et appliquer la tête de soudure dentelée avec une pression sur l'extrémité (13p) du câble d'actionnement (13) pour assurer la pénétration des dents dans la plage en matériau thermoplastique pour déplacer le matériau dans les creux afin d'obtenir au moins un bourrelet (16) de matériau thermoplastique emprisonnant le câble d'actionnement (13) en vue de fixer, par soudage, le câble d'actionnement (13) avec la pièce (12).

2. Procédé de fixation selon la revendication précédente, selon lequel une tension du câble d'actionnement (13) est appliquée lorsque la pression est exercée sur l'extrémité (13p) du câble d'actionnement (13) par la tête de soudure dentelée.

3. Procédé de fixation selon l'une des revendications précédentes, selon lequel est fourni une tête de soudure dentelée (20) comportant un nombre de creux (22) compris entre 1 et 15 et de préférence entre 5 et 8 pour déplacer le matériau en vue de l'obtention d'un nombre correspondant de bourrelets (16) de matériau thermoplastique consécutifs.

4. Procédé de fixation selon l'une des revendications précédentes, selon lequel est fourni une tête de soudure dentelée (20) comportant une série de creux (22) possédant une profondeur comprise entre 0.15 mm et 6 mm pour déplacer le matériau pour obtenir des bourrelets (16) de hauteur déterminée.

5. Procédé de fixation selon l'une des revendications précédentes, selon lequel est fourni une tête de soudure dentelée (20) comportant une série de creux (22) possédant une largeur déterminée pour déplacer le matériau pour obtenir des bourrelets (16) de largeur dépassant de part et d'autre le câble d'actionnement.

6. Procédé de fixation selon l'une des revendications précédentes, selon lequel est fourni une tête de soudure dentelée (20) comportant une série de dents présentant chacune une forme triangulaire pour déplacer le matériau pour obtenir des bourrelets (16) de forme correspondante.

7. Procédé de fixation selon l'une des revendications précédentes, selon lequel une partie du câble d'actionnement (13) est positionnée sur la plage (12b) en matériau thermoplastique, à l'aide de structures de positionnement (19) aménagées sur ladite plage.

8. Procédé de fixation selon l'une des revendications précédentes, selon lequel un soudage par ultrasons assure la fixation du câble d'actionnement (13) à la pièce (12).

9. Procédé de fixation selon l'une des revendications 1 à 7, selon lequel un bouterollage à chaud et par pression assure la fixation du câble d'actionnement (13) à la pièce (12).

10. Procédé de fixation selon l'une des revendications précédentes, selon lequel on fournit en tant que pièce (12), une pièce pivotante faisant partie d'un mécanisme de commande permettant d'orienter la tête distale d'un dispositif médical, cette pièce pivotante (12) étant pourvue de deux plages en matériau thermoplastique pour la fixation chacune d'une extrémité proximale (13p) d'un câble d'actionnement (13).

11. Pièce (12) d'un mécanisme de commande pourvue d'au moins une plage de fixation (12b) en matériau thermoplastique présentant au moins un bourrelet (16) en matériau thermoplastique emprisonnant une partie d'ancrage (13a) d'un câble d'actionnement (13), **caractérisée en ce que** la partie d'ancrage (13a) est enfoncée dans la pièce (12) en étant située en retrait par rapport aux parties (13b, 13c) du câble d'actionnement situées de part et d'autre de la partie d'ancrage (13a).

12. Pièce selon la revendication précédente, selon laquelle elle est pourvue de deux plages (12b) en matériau thermoplastique présentant chacune au moins un bourrelet (16) en matériau thermoplastique emprisonnant chacun une partie d'ancrage (13a) d'un câble d'actionnement (13) enfoncée dans la pièce (12) en étant située en retrait par rapport aux parties (13b, 13c) du câble d'actionnement situées de part et d'autre de la partie d'ancrage (13a).

13. Pièce selon l'une des revendications 11 à 12, selon laquelle elle comporte des bourrelets (16) en matériau thermoplastique emprisonnant le câble d'actionnement (13) et étant situés à proximité de l'entrée d'une rainure de guidage (12m) pour le câble d'actionnement.

14. Pièce selon l'une des revendications 11 à 13, selon laquelle les bourrelets (16) se présentent sous la forme de dentelures successives recouvrant le câble d'actionnement et séparées entre elles par des dégagements (17) dans lesquels affleure le câble d'actionnement.

15. Pièce selon l'une des revendications 11 à 14, selon laquelle la pièce (12) est une pièce pivotante faisant partie d'un mécanisme de commande permettant d'orienter la tête distale d'un dispositif médical.

16. Pièce selon la revendication précédente, selon laquelle elle se présente sous la forme d'un disque ou d'une bague annulaire pour former une poulie d'actionnement.

17. Poignée de commande pour dispositif médical comportant un mécanisme de commande (5) permettant d'orienter la tête distale (4) d'un dispositif médical, le mécanisme de commande comportant une pièce (12) conforme à l'une des revendications 11 à 16.

## Patentansprüche

1. Verfahren zum Befestigen eines Endes eines Betätigungskabels (13), das Teil eines Steuerungsmechanismus (5) ist, wobei das Verfahren die folgenden Schritte umfasst:
- Bereitstellen eines Bauteils (12), das Teil eines Steuerungsmechanismus ist, wobei dieses Bauteil (12) mit mindestens einem Bereich (12b) aus thermoplastischem Material zur Befestigung eines Endes (13p) eines Betätigungskabels (13) versehen ist;
- Bereitstellen eines gezahnten Schweißkopfes (20), der aus abwechselnd durch Vertiefungen getrennten Zähnen gebildet ist;
- Positionieren des Endes (13p) eines Betätigungskabels (13) auf einem Bereich aus thermoplastischem Material des Bauteils;
- Sicherstellen der Erwärmung des Bereichs des Bauteils und Anlegen des gezahnten Schweißkopfes unter Druck auf das Ende (13p) des Betätigungskabels (13), um das Eindringen der Zähne in den Bereich aus thermoplastischem Material sicherzustellen, um das Material in die Vertiefungen zu bewegen, um mindestens eine Wulst (16) aus thermoplastischem Material zu erhalten, die das Betätigungskabel (13) einschließt, um das Betätigungskabel (13) durch Schweißen an dem Bauteil (12) zu befestigen.

2. Verfahren zum Befestigen gemäß dem vorstehenden Anspruch, wobei eine Spannung des Betätigungskabels (13) aufgebracht wird, wenn durch den gezahnten Schweißkopf Druck auf das Ende (13p) des Betätigungskabels (13) ausgeübt wird.

3. Verfahren zum Befestigen nach einem der vorstehenden Ansprüche, wobei ein gezahnter Schweißkopf (20), der eine Anzahl von Vertiefungen (22) zwischen 1 und 15 und vorzugsweise zwischen 5 und 8 zum Bewegen des Materials aufweist, bereitgestellt ist, um eine entsprechende Anzahl aufeinanderfolgender Wülste (16) aus thermoplastischem Material zu erhalten.

4. Verfahren zum Befestigen nach einem der vorstehenden Ansprüche, wobei ein gezahnter Schweißkopf (20) bereitgestellt ist, der eine Reihe von Vertiefungen (22) mit einer Tiefe zwischen 0,15 mm und 6 mm aufweist, um das Material zu bewegen, um Wülste (16) einer bestimmten Höhe zu erhalten.

5. Verfahren zum Befestigen nach einem der vorstehenden Ansprüche, wobei ein gezahnter Schweißkopf (20) bereitgestellt ist, der eine Reihe von Vertiefungen (22) mit einer bestimmten Breite aufweist, um das Material zu bewegen, um Wülste (16) zu erhalten, deren Breite auf beiden Seiten über das Betätigungskabel hinausragt.

6. Verfahren zum Befestigen nach einem der vorstehenden Ansprüche, wobei ein gezahnter Schweißkopf (20) bereitgestellt ist, der eine Reihe von Zähnen aufweist, die jeweils eine dreieckige Form aufweisen, um das Material zu bewegen, um Wülste (16) von entsprechender Form zu erhalten.

7. Verfahren zum Befestigen nach einem der vorstehenden Ansprüche, wobei ein Teil des Betätigungskabels (13) auf dem Bereich (12b) aus thermoplastischem Material unter Verwendung von auf diesem Bereich angeordneten Positionierstrukturen (19) positioniert wird.

8. Verfahren zum Befestigen nach einem der vorstehenden Ansprüche, wobei die Befestigung des Betätigungskabels (13) am Bauteil (12) durch Ultraschallschweißen gewährleistet wird.

9. Verfahren zum Befestigen nach einem der Ansprüche 1 bis 7, wobei durch Heiß- und Drucknieten die Befestigung des Betätigungskabels (13) am Bauteil (12) gewährleistet wird.

10. Verfahren zum Befestigen nach einem der vorstehenden Ansprüche, wobei als Bauteil (12) ein schwenkbares Bauteil bereitgestellt ist, das Teil eines Steuerungsmechanismus ist, der das Orientieren des distalen Kopfes einer medizinischen Vorrichtung ermöglicht, wobei dieses schwenkbare Bauteil (12) mit zwei Bereichen aus thermoplastischem Material zur Befestigung jeweils eines proximalen Endes (13p) eines Betätigungskabels (13) versehen ist.

11. Bauteil (12) eines Steuerungsmechanismus, der mit mindestens einem Befestigungsbereich (12b) aus thermoplastischem Material versehen ist, der mindestens eine Wulst (16) aus thermoplastischem Material aufweist, die einen Verankerungsteil (13a) eines Betätigungskabels (13) einschließt,
**dadurch gekennzeichnet, dass** das Verankerungsteil (13a) in das Bauteil (12) eingeschoben wird, wobei es relativ zu den Teilen (13b, 13c) des Betätigungskabels, die sich auf beiden Seiten des Verankerungsteils (13a) befinden, zurück versetzt angeordnet ist.

12. Bauteil nach dem vorstehenden Anspruch, wobei es mit zwei Bereichen (12b) aus thermoplastischem Material versehen ist, die jeweils mindestens eine Wulst (16) aus thermoplastischem Material aufweisen, die jeweils einen Verankerungsteil (13a) eines Betätigungskabels (13) einschließt, das in das Bauteil (12) eingeschoben ist, wobei es relativ zu den Teilen (13b, 13c) des Betätigungskabels, die sich auf beiden Seiten des Verankerungsteils (13a) befinden, zurück versetzt angeordnet ist.

13. Bauteil nach einem der Ansprüche 11 bis 12, wobei es Wülste (16) aus thermoplastischem Material aufweist, die das Betätigungskabel (13) einschließen und sich in der Nähe des Eintritts einer Führungsnut (12m) für das Betätigungskabel befinden.

14. Bauteil nach einem der Ansprüche 11 bis 13, wobei die Wülste (16) die Form aufeinanderfolgender, das Betätigungskabel bedeckender Zähne aufweisen und durch Aussparungen (17) voneinander getrennt sind, in denen das Betätigungskabel bündig liegt.

15. Bauteil nach einem der Ansprüche 11 bis 14, wobei das Bauteil (12) ein schwenkbares Bauteil ist, das Teil eines Steuerungsmechanismus ist, der das Orientieren des distalen Kopfes einer medizinischen Vorrichtung ermöglicht.

16. Bauteil nach dem vorstehenden Anspruch, wobei es die Form einer Scheibe oder eines kreisförmigen Rings aufweist, um eine Betätigungsrolle zu bilden.

17. Steuerungsgriff für eine medizinische Vorrichtung, das einen Steuerungsmechanismus (5) zum Orientieren des distalen Kopfes (4) einer medizinischen Vorrichtung aufweist, wobei der Steuerungsmechanismus ein Bauteil (12) gemäß einem der Ansprüche 11 bis 16 aufweist.

## Claims

1. Method for securing one end of an actuation cable (13) forming part of a control mechanism (5), the method comprising the following steps:
- providing a part (12) forming part of a control mechanism, this part (12) being provided with at least one region (12b) made of thermoplastic material for securing one end (13p) of an actuation cable (13);
- providing a serrated welding head (20) formed of teeth alternately separated by hollows;
- positioning the end (13p) of an actuation cable (13) on a region made of thermoplastic material of the part;
- heating the region of the part and applying the serrated welding head with pressure on the end (13p) of the actuation cable (13) to ensure penetration of the teeth into the region made of thermoplastic material to move the material into the hollows in order to obtain at least one bead (16) of thermoplastic material trapping the actuation cable (13) with a view to fix, by welding, the actuation cable (13) with the part (12).

2. Securing method according to the preceding claim, wherein tension of the actuation cable (13) is applied when pressure is exerted on the end (13p) of the actuation cable (13) by the serrated welding head.

3. Securing method according to either of the preceding claims, wherein a serrated welding head (20) is provided having a number of hollows (22) of between 1 and 15 and preferably between 5 and 8 for moving the material with a view to obtain a corresponding number of consecutive beads (16) of thermoplastic material.

4. Securing method according to any of the preceding claims, wherein a serrated welding head (20) is provided having a series of hollows (22) of a depth of between 0.15 mm and 6 mm for moving the material in order to obtain beads (16) of predetermined height.

5. Securing method according to any of the preceding claims, wherein a serrated welding head (20) is provided having a series of hollows (22) of a given width for moving the material in order to obtain beads (16) of a width exceeding the actuation cable on either side.

6. Securing method according to any of the preceding claims, wherein a serrated welding head (20) is provided having a series of teeth each having a triangular shape for moving the material in order to obtain correspondingly shaped beads (16).

7. Securing method according to any of the preceding claims, wherein part of the actuation cable (13) is positioned on the region (12b) made of thermoplastic material by means of positioning structures (19) provided on the region.

8. Securing method according to any of the preceding claims, wherein ultrasonic welding secures the actuation cable (13) to the part (12).

9. Securing method according to any of claims 1 to 7, wherein the actuation cable (13) is secured to the part (12) by hot and pressure riveting.

10. Securing method according to any of the preceding claims, wherein a pivoting part forming part of a control mechanism for orienting the distal head of a medical device is provided as a part (12), this pivoting part (12) being provided with two regions made of thermoplastic material for the securing of one proximal end (13p) each of an actuation cable (13).

11. Part (12) of a control mechanism provided with at least one securing region (12b) made of thermoplastic material having at least one bead (16) made of thermoplastic material enclosing an anchoring part (13a) of an actuation cable (13), **characterized in that** the anchoring part (13a) is pressed into the part (12) and is set back from the parts (13b, 13c) of the actuation cable located on either side of the anchoring part (13a).

12. Part according to the preceding claim, wherein it is provided with two regions (12b) made of thermoplastic material, each having at least one bead (16) made of thermoplastic material, each trapping an anchoring part (13a) of an actuation cable (13) inserted into the part (12) and set back with respect to the parts (13b, 13c) of the actuation cable located on either side of the anchoring part (13a).

13. Part according to any of claims 11 to 12, wherein it comprises beads (16) made of thermoplastic material trapping the actuation cable (13) and being located close to the entrance to a guide groove (12m) for the actuation cable.

14. Part according to any of claims 11 to 13, wherein the beads (16) are in the form of successive serrations covering the actuation cable and separated from one another by hollows (17) in which the actuation cable is flush.

15. Part according to any of claims 11 to 14, wherein the part (12) is a pivoting part forming part of a control mechanism for orienting the distal head of a medical device.

16. Part according to the preceding claim, wherein it is in the form of a disc or an annular ring to form an actuation pulley.

17. Control handle for a medical device comprising a control mechanism (5) for orienting the distal head (4) of a medical device, the control mechanism comprising a part (12) in accordance with any of claims 11 to 16.
